# FASCICULE DE BREVET EUROPEEN

(11) **EP 0 541 467 B1**
(45) Date de publication et mention de la délivrance du brevet: **25.06.1997**
(21) Numéro de dépôt: 92420399.5
(22) Date de dépôt: 05.11.1992
(51) Int. Cl.: C07C 235/10, C07H 15/04, C07H 13/04, C07H 15/12

(54) **Utilisation des alkyldiamides amphiphiles à double tête de sucre pour obtenir une composition tensioactive**
Verwendung von Alkyldiamiden mit zwei Zucker-Gruppen als amphiphilische Verbindungen zur Herstellung von Tensiden
Utilisation of amphiphilic alkyldiamides compounds with two sugar groups for the preparation of surface-active compositions

(30) Priorité: 05.11.1991 FR 9113851
(43) Date de publication de la demande: 12.05.1993
(73) Titulaire: STEPAN EUROPE, 38340 Voreppe (FR)
(72) Inventeur: Garelli-Calvet, Rachel, F-31380 MONTASTRUC LA CONSEILLERE (FR); Brisset, Florence, F-31130 Balma (FR); Rico, Isabelle, F-31520 Ramonville (FR); Godefroy, Lionel, F-38430 Moirans (FR); LATTES, ARMAND, F-31520 Ramonville (FR)
(74) Mandataire: Guerre, Dominique

(56) Documents cités:
- EP-A- 0 312 087
- EP-A- 0 447 064
- FR-A- 2 235 113
- US-A- 3 855 290
- ANALYTICAL BIOCHEMISTRY vol. 130 , 1983 pages 485 - 490 L.H. HJELMELAND ET AL 'A NEW CLASS OF NONIONIC DETERGENTS WITH A GLUCONAMIDE POLAR GROUP'
- ANGEWANDTE CHEMIE. INTERNATIONAL EDITION vol. 23 , 1984 , WEINHEIM DE pages 100 - 113 J.-H. HINRICH ET AL. 'ROUTES TO FUNCTIONAL VESICLE MEMBRANES WITHOUT PROTEINS'
- STARCH vol. 33, no. 6 , Juin 1981 , WEINHEIM pages 202 - 208 W.N. EMMERLING ET AL 'PRÄPARATIVE METHODEN ZUR HERSTELLUNG VON HöHEREN MALTOOLIGOMEREN UND IHRE VERKNÜPFUNG MIT ALIPHATISCHEN DIAMINEN'
- ANALYTICAL BIOCHEMISTRY, vol. 130, 1983, pages 485-490, L.H. HJELMELAND et al, "A new class of nonionic detergents with a fluconamide polar group"
- STARCH, vol. 33, no. 6, juin 1981, Weinheim, DE, pages 202-208, W.N. EMMERLING et al, "Präparative Methoden zur Herstellung von höheren Maltooligomeren und ihre Verknüpfung mit aliphatischen Diaminen"

## Description

L'invention se rapporte à de nouveaux composés dits amphiphiles ou bolaformes, présentant des propriétés tensio-actives, et utilisables notamment comme agents vésiculaires.

Par composés bolaamphiphiles ou bolaformes, on entent tout composé comportant :
- une chaîne carbonée relativement longue, linéaire ou ramifiée, et hydrophobe, avec éventuellement des hétéroa- tomes
- et deux parties polaires ou têtes hydrophiles, disposées et liées par covalence respectivement aux deux extrémités de la chaîne carbonée.

La terminologie "bola" provient d'une arme d'Amérique du Sud, constituée par deux boules reliées par une corde ou une lanière de cuir.

Différents composés bolaformes naturels sont déjà connus, et constituent la membrane lipidique de bactéries appelées archéobactéries; cf T.D. BROCK, La Recherche, 1988,19(198), 478.

On connaît des composés bolaformes, tensio-actifs, qui permettent la formation d'agrégats de type micelles, cylindres, ou à concentration plus élevée, de cristaux liquides lyotropes (phases hexagonales, lamellaires). Ces différents agrégats sont obtenus, par des processus encore mal connus, à partir d'au moins une bicouche du composé bolaforme, elle-même obtenue à partir d'une solution biphasique eau/composé bolaforme, souvent avec un apport d'énergie, par exemple sonication.

A titre d'exemple, l'article de J. H. FUHRHOP et al, Angewandte Chemie, International Edition, vol. 23, 1984, 100-113, décrit différentes membranes vésiculaires obtenues à partir de composés bolaamphiphiles.

Différents composés bolaformes ont été déjà obtenus et étudiés, à savoir :
- des composés avec des têtes ammoniums quaternaires, (cf F.M. MENGER et S. WRENN, J Phys Chem, 1976, 80, (24), 2651), éventuellement avec deux chaînes carbonées relativement longues entre les deux têtes hydrophiles, (cf JH FUHRHOP, et al J. Am. Chem. Soc, 1986, 108, 1785)
- des composés avec des têtes anioniques, identiques ou non, du type sulfate ou carboxylate, séparées par une ou deux chaînes hydrocarbonées (cf JH FUHRHOP, et al, J. Am. Chem. Soc, 1986, 108, 1785)
- des composés avec des têtes non ioniques, identiques ou non du type polyol (cf par exemple C. TSCHIERSKE et H. ZASCHKE, J. Chem. Soc, Chem. Commun, 1990, 1013)
- et enfin des composés symétriques avec deux têtes de composés dérivées de sucre, reliées entre elles par deux chaînes hydrocarbonées, chaque tête consistant en un résidu d'un thioglucoside ou d'un thiogalactoside, lié par l'atome de soufre au squelette comprenant les deux chaînes hydrocarbonées, (cf JH FUHRHOP, et al, J. Am. Chem. Soc, 1986, 108, 1785).

La synthèse de ces composés fait intervenir plusieurs étapes successives, avec des rendements relativement faibles pour certaines d'entre elles, ce qui rend de tels composés inadaptés à une production industrielle.

Le document Analytical Biochemistry, vol. 130, 1983, 485-490, de L. H. HJELMELAND, décrit la préparation de composés bolaamphiphiles comprenant deux têtes hydrophiles et une chaîne hydrophobe consistant en un résidu de l'acide cholique, désoxycholique ou octanoïque. La chaîne hydrophobe greffée sur le groupe qui relie les deux têtes est dégagée de cette dernière et est très mobile, conférant aux composés des propriétés de détergents.

Différents documents ont déjà décrit des composés comprenant une chaîne carbonée relativement longue, avec deux parties sucres ou dérivées de sucre, liées par covalence respectivement aux deux extrémités de la chaîne carbonée, mais sans mettre en évidence de quelconques propriétés tensio-actives attachées à ces composés :
- le document EP-A-0 447 064 a décrit, à titre d'agent adoucissant dans une composition cosmétique, des N-N'- dilactobionamidoalcanes comprenant :
   ^{*} une chaîne carbonée présentant de 2 à 14 atomes de carbone,
   ^{*} deux résidus osidiques, comprenant chacun de 1 à 5 atomes de carbone, liés par leurs fonctions aldéhyde ou cétone, respectivement aux deux extrémités de la chaîne hydrophiles, (cf JH FUHRHOP, et al J. Am. Chem. Soc, 1986, 108,1785)
- des composés avec des têtes anioniques, identiques ou non, du type sulfate ou carboxylate, séparées par une ou deux chaînes hydrocarbonées (cf JH FUHRHOP, et al, J. Am. Chem. Soc, 1986, 108, 1785)
- des composés avec des têtes non ioniques, identiques ou non du type polyol (cf par exemple C. TSCHIERSKE et H. ZASCHKE, J. Chem. Soc, Chem. Commun, 1990, 1013)
- et enfin des composés symétriques avec deux têtes de composés dérivées de sucre, reliées entre elles par deux chaînes hydrocarbonées, chaque tête consistant en un résidu d'un thioglucoside ou d'un thiogalactoside, lié par l'atome de soufre au squelette comprenant les deux chaînes hydrocarbonées, (cf JH FUHRHOP, et al, J. Am. Chem. Soc, 1986, 108, 1785).

La synthèse de ces composés fait intervenir plusieurs étapes successives, avec des rendements relativement faibles pour certaines d'entre elles, ce qui rend de tels composés inadaptés à une production industrielle.

Le document Analytical Biochemistry, vol. 130, 1983, 485-490, de L. H. HJELMELAND, décrit la préparation de composés bolaamphiphiles comprenant deux têtes hydrophiles et une chaîne hydrophobe consistant en un résidu de l'acide cholique, désoxycholique ou octanoïque. La chaîne hydrophobe greffée sur le groupe qui relie les deux têtes est dégagée de cette dernière et est très mobile, conférant aux composés des propriétés de détergents.

Différents documents ont déjà décrit des composés comprenant une chaîne carbonée relativement longue, avec deux parties sucres ou dérivées de sucre, liées par covalence respectivement aux deux extrémités de la chaîne aliphatiques; il s'agit d'une étude scientifique, n'ayant pas mis en évidence de propriétés particulièrement utiles des composés ainsi préparés.

La présente invention a donc pour objet de nouveaux composés bolaformes, à deux têtes de sucre ou dérivées de sucre, pouvant être obtenus en une, voire deux étapes, au maximum avec des rendements acceptables, et pouvant être de ce fait produits de manière industrielle.

Une composition selon la présente invention comporte un composé bolaforme répondant à la formule générale suivante:
RAEAR

dans laquelle :
- E représente une chaîne carbonée, comportant au moins six atomes de carbone, interrompue éventuellement par au moins un groupement aromatique, ou hétérocyclique, ou fonctionnel
- A est un groupe fonctionnel de liaison
- R est un résidu d'un glucide réducteur à chaîne linéaire ou cyclisée, ou d'un dérivé dudit glucide réducteur, lié directement ou indirectement (par exemple par une chaîne alkyle) par une de ses fonctions aldéhyde ou cétone, ou dérivée (par exemple amide), au groupe fonctionnel A.

Outre leurs propriétés tensio-actives, et notamment vésiculaires, les composés bolaformes selon la présente invention présentent l'avantage déterminant de pouvoir être synthétisés directement par condensation entre une forme acide du monosaccharide (par exemple acide gluconique), ou du polysaccharide (par exemple acide lactobionique), sans passer par la forme lactone ou ester interne du même saccharide, comme préconisé par certains documents de l'art antérieur.

Un des intérêts principaux des compositions de la présente invention est apporté par leur comportement en solution aqueuse, et concerne leur application comme agent tensioactif. Le domaine de ces applications est d'autant plus large que ces compositions ne présentent pas ou peu de toxicité, ce qui permet d'envisager leur emploi en cosmétique, pharmacie, etc...

Leur comportement en solution aqueuse leur confère des propriétés particulièrement intéressantes, en ce qu'ils peuvent former directement des structures vésiculaires.

Cette propriété permet d'utiliser les composés selon l'invention comme agents d'encapsulation, mais aussi pour l'extraction des protéines membranaires, l'immunologie, etc...

En outre, l'invention permet d'apporter des composés bolaformes possédant des propriétés d'isomérie optique, les deux parties hydrophiles étant chirales. Les composés de l'invention peuvent alors être utilisés comme inducteur d'asymétrie.

Un composé bolaforme avantageux de la présente invention comprend une chaîne carbonée hydrophobe, linéaire et saturée.

Avantageusement, le nombre d'atomes de carbone de la chaîne carbonée du composé bolaforme est compris entre 6 et 24 atomes de carbone, et de préférence compris entre 6 et 14.

Le groupe fonctionnel A du composé bolaforme est notamment choisi dans le groupe comprenant les fonctions :
0, OCO, S, NH, NHCO,

les fonctions OCO comprenant les formules développées : 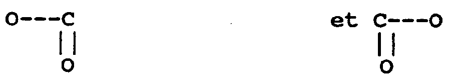 et NHCO comprenant les formules développées :

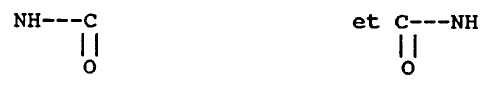

De préférence, le groupe fonctionnel A est la fonction amine NH.

Le glucide réducteur du résidu R du composé bolaforme est un monosaccharide ou un polysaccharide.

Parmi les monosaccharides préférés, le glucide réducteur est choisi dans le groupe comprenant le glucose et le galactose.

Parmi les polysaccharides, les disaccharides sont préférés, et notamment choisis dans le groupe comprenant le lactose, le cellobiose et le maltose.

Une autre famille de composés bolaformes selon la présente invention est obtenue, en choisissant :
- la fonction NHCO et/ou CONH comme groupe fonctionnel de liaison A
- et un gluconamide comme dérivé d'une glucide réducteur, lié par sa fonction amide, par une chaîne alkyle comportant de deux à six atomes de carbone, au groupe fonctionnel de liaison A.

Ces composés ont pour formule développée :

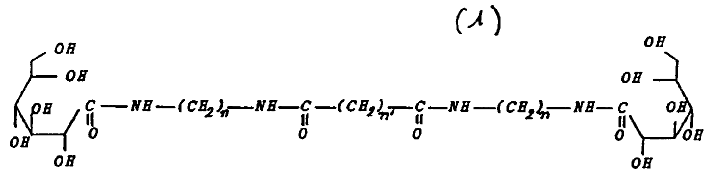

Ils peuvent être obtenus par réaction directe de l'acide gluconique, d'un acide akyldioïque, et d'un diaminoalcane.

Une autre famille de composés bolaformes selon la présente invention est obtenue, en choisissant:
- la fonction NH comme groupe fonctionnel de liaison A
- une chaîne carbonée E interrompue par un ou plusieurs groupements fonctionnels NH
- et un glucide réducteur constitué par un disaccharide, par exemple du lactose.

Il s'agit à titre d'exemple de cette famille des bislactobionamidospermidines et bislactobionamidospermines.

Une autre famille de composés bolaformes selon la présente invention est obtenue, en choisissant :
la fonction NH comme groupe fonctionnel de liaison A

- une chaîne E interrompue par une ou plusieurs fonctions O
- et un glucide réducteur constitué par un monosaccharide tel que glucose, ou un dissacharide, par exemple du lactose.

Il s'agit à titre d'exemple de cette famille des bisgluconamidodioxo ou trioxoalcanes et des bislactobionamidodioxo ou trioxoalcanes.

Une autre famille de composés bolaformes selon la présente invention est obtenue, en choisissant :
- la fonction NH comme groupe fonctionnel de liaison A
- et un glucide réducteur constitué par un monosaccharide ou polysaccharide, comportant un hétérocycle oxygéné, ou un dérivé (par exemple aminé) dudit glucide.

Il s'agit à titre d'exemple de cette famille des bisaminolactose alcanes, ou des bisglucosaminoalcanes.

L'invention a également pour objet un procédé pour préparer de tels composés amphiphiles.

Ce procédé consiste à faire réagir directement une forme réactive, oxydée, ou réduite, ou aminée du glucide réducteur du résidu R, avec un produit répondant à la formule B E B, E étant la chaîne carbonée hydrophobe décrite ci-dessus, et B correspondant au groupe fonctionnel A avant condensation avec la forme réactive du glucide.

La réaction est de préférence réalisée dans un solvant alcoolique, et notamment dans le méthanol.

Selon le substrat de départ, la réaction peut avoir lieu à température ambiante sous agitation, ou bien le milieu réactionnel doit être chauffé et notamment à la température de reflux du solvant.

Selon les conditions opératoires, le meilleur rendement de réaction est obtenu pour des temps de réaction compris entre 12 et 48 heures, et de préférence de 24 heures.

Pour préparer un composé de l'invention, la forme oxydée de départ choisie peut être la forme lactone ou la forme acide; on choisira préférentiellement la forme acide.

Ainsi, pour préparer un composé pour lequel R est un résidu du glucose, on pourra choisir la gluconolactone pour réagir avec le produit B E B.

Pour préparer un composé pour lequel R est un résidu du lactose, on pourra choisir l'acide lactobionique pour réagir avec le produit B E B.

Les composés bolaformes de l'invention, une application et un procédé de préparation sont à présent détaillés avec les exemples 1 à 12 suivants.

Dans les exemples suivants, les microanalyses des composés préparés ont été réalisées sur un appareil TECH-NICON.

Les analyses en RMN ont été réalisées sur un spectrophotomètre BRUCKER AC200 à 200,13 MHz pour la RMN ¹H et à 50,32 MHz pour la _{RMN} ¹³_{C}.

Les RMN ¹ H et ¹³C sont réalisées dans le DMSO.

### Exemple 1 : 1.12-digluconamidododécane

A une solution contenant 1 g de gluconolactone et 50 ml de méthanol, on ajoute 0,56 g de 1,12-diaminododécane. Le milieu réactionnel est agité 24 heures à température ambiante. Le dérivé bolaamphiphile 1,12-digluconamidododécane précipite dans le milieu et est filtré. Le solide obtenu est séché puis lyophilisé.

On obtient 1,38 g de produit solide blanc dont les analyses montrent qu'il s'agit du composé de formule :

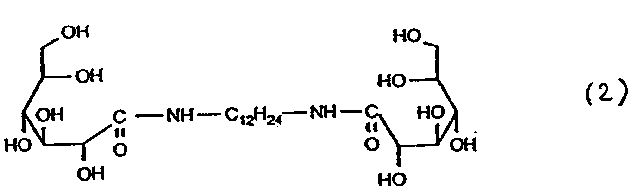

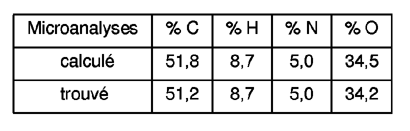

### Exemple 2 : 1.10-digluconamidodécane

On procède de la même façon que dans l'exemple 2, en utilisant 1 g de gluconolactone et 0,48 g de 1,10-diamino- décane.

On récupère 1,28 g de produit dont les analyses montrent qu'il s'agit du produit de formule :

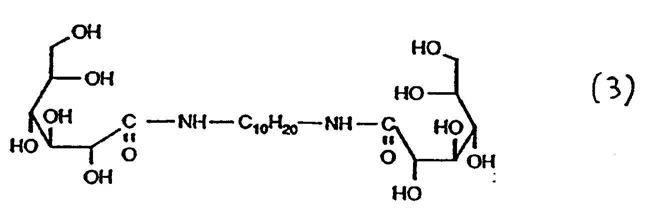

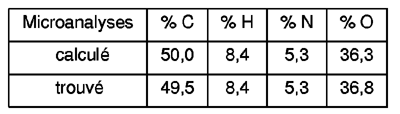

### Exemple 3 : 1.9-digluconamidononane

On procède de la même façon que dans les exemples précédents en utilisant 1 g de gluconolactone et 0,45 g de 1,9-diaminononane.

On récupère 1,30 g de produit dont les analyses montrent qu'il s'agit du produit de formule :

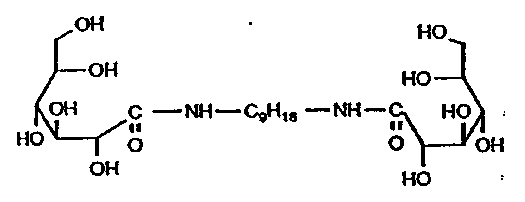

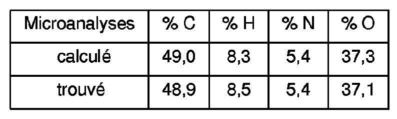

### Exemple 4 : 1,8-digluconamidooctane

On procède de la même façon que dans les exemples précédents en utilisant 1 g de gluconolactone et 0,41 g de 1,8-diaminooctane.

On récupère 1,20 g de produit dont les analyses montrent qu'il s'agit du produit de formule :

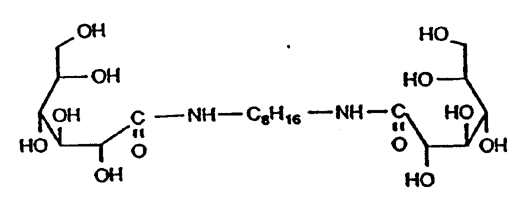

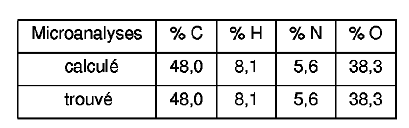

### Exemple 5 : 1,7-digluconamidoheptane

On procède de la même façon que dans les exemples précédents en utilisant 1 g de gluconolactone et 0,37 g de 1,7-diaminoheptane.

On récupère 1,07 g de produit dont les analyses montrent qu'il s'agit du produit de formule :

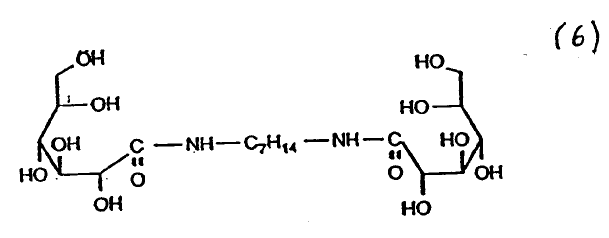

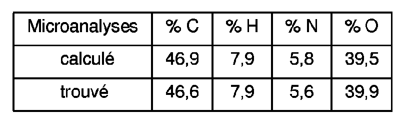

### Exemple 6 : 1.6-digluconamidohexane

On procède de la même façon que dans les exemples précédents en utilisant 1 g de gluconolactone et 0,33 g de 1,6-diaminohexane.

On récupère 1,18 g de produit dont les analyses montrent qu'il s'agit du produit de formule :

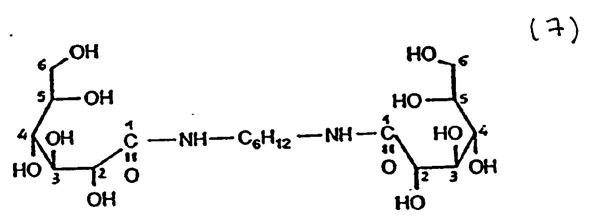

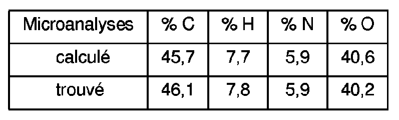

RMN:
CHAINE ALKYLE : -NH- 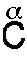 H₂- 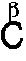 H₂- 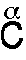 H₂- 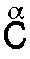 H₂- 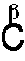 H₂- 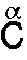 H₂-NH-
RMN¹³C :
CHAINE ALKYLE : CH₂(25,98) ; 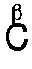 H₂(29,01) ; 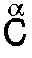 H₂(38,12)
PARTIE SUCRE : C₆
   C₂, C₃, C₄, C₅ (70,04 ; 71,40 ; 72,33 ; 73,55)
   C₁ (172,18)
RMN¹H :
   1,25 ppm (4p, m, 2CH₂)
   1,41 ppm (4^{p}, m, 2 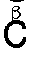 H₂)
   3-6 ppm (26p, m, CHOH, CH₂0H, CH₂-NH-C=O)
   7,60 ppm (2p,t,2NH)

### Exemple 7 : 1.12-dilactobionamidododécane :

A une solution d'acide lactobionique (1 g) dans 100 ml de méthanol, on ajoute 0,26 g de 1,12-diaminododécane. Le mélange réactionnel est agité pendant 24 heures au reflux du méthanol. Le solvant est évaporé sous vide, puis le produit est lyophilisé.

On obtient 1,16 g de produit solide dont les analyses montrent qu'il s'agit du composé de formule :

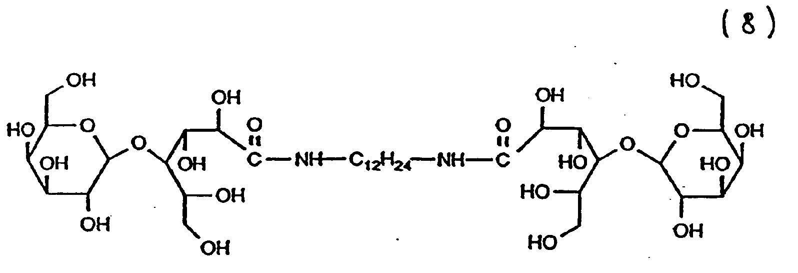

Les microanalyses des dérivés du lactose ne sont pas indiquées car les produits sont très hygroscopiques.

### Exemple 8 : 1.10-dilactobionamidodécane

On procède de la même façon que dans l'exemple 7 en utilisant 1 g d'acide lactobionique et 0,23 g de 1,10-diami- nodécane.

On récupère 1,10 g de produit dont les analyses montrent qu'il s'agit du produit de formule :

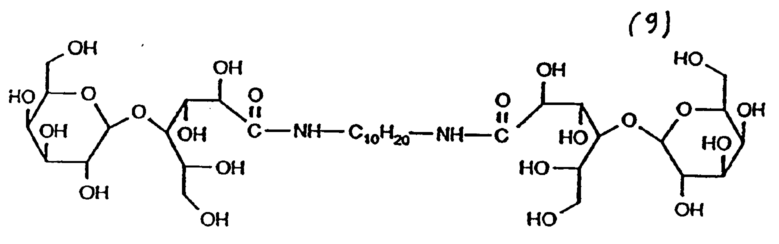

### Exemple 9 : 1.9-dilactobionamidononane

On procède de la même façon que dans les exemples précédents en utilisant 1 g d'acide lactobionique et 0,21 g de 1,9-diaminononane.

On récupère 1,08 g de produit dont les analyses montrent qu'il s'agit du produit de formule : 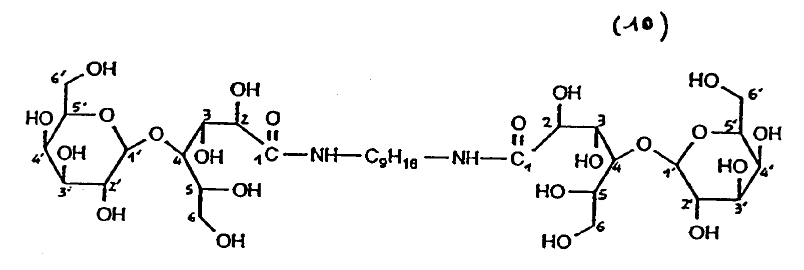 RMN:
CHAINE ALKYLE: -NH- 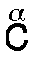 H₂- 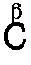 H₂- 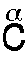 H₂- 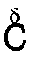 H₂- 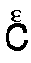 H₂- 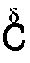 H₂- 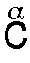 H₂- 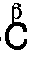 H₂- 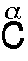 H₂-NH-
RMN¹³C :
CHAINE ALKYLE : 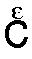 H₂(26,50) ; 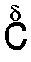 H2(28,72) ; 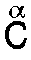 H2(28,93) ; 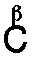 H₂(29,14) ; 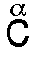 H2(38,16)
PARTIE SUCRE :
   C₆ et C₆, (60,59 ; 62,27)
   C₁ (171,97)
   C₁' (104,54)
RMN¹H :
   1,24 ppm (10p, m, 5CH₂)
   3-6 ppm (46p, m, CHOH, CH₂0H, CH₂-NH-C=O, CH-O)
   7,57 ppm (2p,t,2NH)

### Exemple 10 : 1.8-dilactobionamidooctane

On procède de la même façon que dans les exemples précédents en utilisant 1 g d'acide lactobionique et 0,19 g de 1,8-diaminooctane.

On récupère 1,06 g de produit dont les analyses montrent qu'il s'agit du produit de formule :

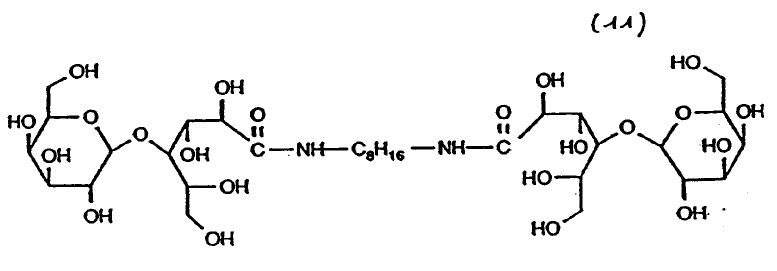

### Exemple 11

Les propriétés tensioactives en solution aqueuse du 1,8-digluconamidooctane et du 1,8-dilactobionamidooctane préparés respectivement selon les exemples 4 et 10 peuvent être mesurées par une technique usuelle de tensiométrie en utilisant un tensiomètre Tensimat N3 (Prolabo, FRANCE). Le mobile de mesure est un étrier fourni avec l'appareil formé d'un fil de platine de 0,1 mm de diamètre et long de 2 cm (écart entre les deux branches de l'étrier). Les résultats sont illustrés par les tableaux I et II indiquant les tensions superficielles à concentration variable en dérivé bolaamphiphile (dans le Tableau I avec le 1,8-digluconamidooctane et dans le Tableau Il avec le 1,8-dilactobionamidooctane).

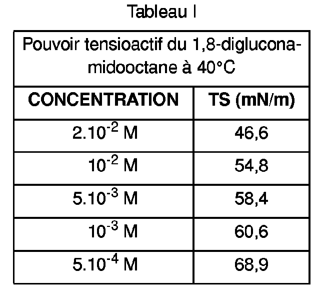

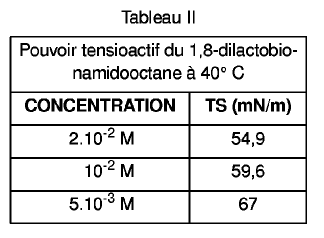

### Exemple 12

L'examen par diffusion de la lumière (à l'aide d'un appareil COULTER mode N4MD) de solutions de 1,8-digluconamidooctane et de 1,8-dilactobionamidooctane dans une gamme de concentrations pouvant aller de 10⁻³ M à 5.10-² M permet de mettre en évidence l'existence d'agrégats dune taille maximale de 1000 nm.

Une étude par microscopie électronique indique que ces agrégats sont des vésicules unilamellaires. Le protocole de cette étude peut être brièvement décrit ainsi : quelques gouttes de la solution de bolaamphiphile (qui peut ou non être soniquée) sont appliquées sur une grille de cuivre (150 mesh) recouverte d'un film de carbone. Le dépôt est ensuite desséché. Une solution à 2 % d'acétate d'uranyle dans l'eau est ensuite déposée de la même manière sur la grille.

Les photographies de ces échantillons obtenues à l'aide d'un microscope électronique (Phillips EM 301) montrent :
- pour le 1,8-digluconamidooctane dune concentration de 0,007 mole/I,
   des vésicules de 165 nm, de 325 nm (grandissement x 42000)
- pour le 1,8-dilactobionamidooctane dune concentration de 0,02 mole/I,
   des vésicules de 125 nm, de 280 nm, de 375 nm (grandissement x 32000).

D'autres composés tensioactifs bolaamphiphiles ou bolaformes, à deux têtes dérivées de sucre, ont été synthétisés, à savoir les cinq familles chimiques suivantes :
1- Des bis ou digluconamidoalcanes et les bis ou dilactobionamidoalcanes, préparés selon un procédé différent de celui des exemples 1 à 10.
2- Les bis ou dilactobionamidospermidine et spermine.
3- Les bis ou digluconamidodioxo- ou trioxoalcanes et les bis ou dilactobionamidodioxo- ou trioxoalcanes.
4- Les bis ou diaminolactose alcanes.
5- Les bis ou diglucosaminoalcanes.

Ces familles de tensio-actifs bolaformes ont été synthétisées selon un protocole en une seule étape, sans protection préalable des parties sucres.

### 1 - Les bisgluconamido- et les bislactobionamidoalcanes

### a) Les bisgluconamidoalcanes

La méthode de synthèse consiste à faire réagir un équivalent de diamine à longue chaîne alkyle avec deux équivalents d'acide gluconique dans le méthanol suivant le schéma réactionnel suivant :

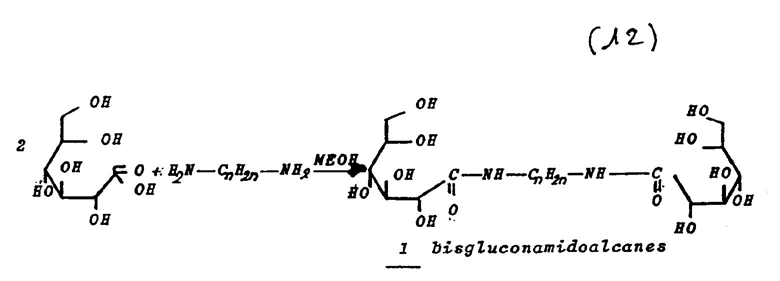

Le milieu réactionnel est maintenu sous agitation à température ambiante pendant 24 heures. Le produit de la réaction précipite. Il est récupéré par filtration et lavé à chaud avec du méthanol pour éliminer les traces de diamine qui éventuellement n'auraient pas réagi.

Les rendements, en produits isolés, sont rassemblés dans le tableau suivant :

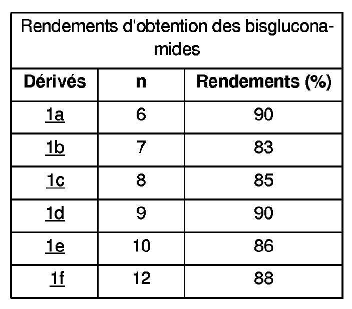

Les produits obtenus sont sous forme de poudre blanche ; ils ont été caractérisés par spectrométrie de masse, RMN du H¹ et ¹³C et microanalyse.

### b) Les bislactobionamidoalcanes

Les bislactobionamidoalcanes sont synthétisés à partir de deux équivalents d'acide lactobiolnique et d'un équivalent de diamine à longue chaîne alcane dans le méthanol suivant le schéma réactionnel suivant :

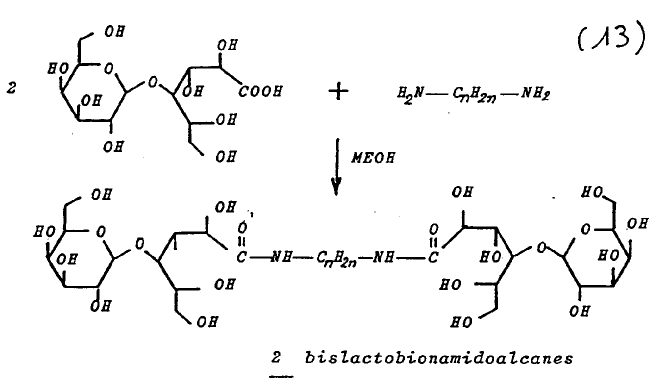

La diamine réagit sur la lactone formée in situ dans le méthanol. Le milieu réactionnel est maintenu sous agitation pendant 24 heures au reflux du méthanol.

Le produit plus hydrophile ne précipite pas. on évapore donc le solvant sous vide et le produit est lyophilisé.

Les rendements de la réaction sont quantitatifs. Ils sont rassemblés dans le tableau suivant :

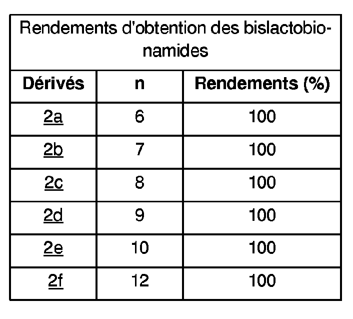

Les dérivés obtenus ont été caractérisés par microanalyse, RMN H¹, ¹³C et spectrométrie de masse.

### 2 - Les bislactobionamidospermidine et les bislactobionamidospermine

Ces composés bolaformes ont été synthétisés à partir de diamines biologiques, la spermidine et la spermine, de formules respectives suivantes :

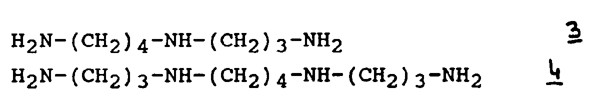

Les bislactobionamidospermidine 3 et les bislactobionamidospermine 4 sont synthétisés à partir de 2 équivalents d'acide lactobionique et d'un équivalent de diamine, dans un mélange eau/propanol-2 ou méthanol. La réaction se fait pendant 24 heures à température ambiante. On évapore alors le solvant sous vide et les produits sont lyophilisés.

Les rendements des réactions ainsi que les modes opératoires sont réunis dans le tableau suivant :

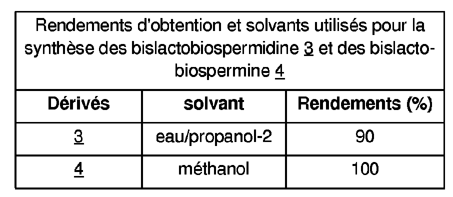

Ces dérivés se présentent sous forme de poudre blanche hydroscopique. Ils ont été caractérisés par RMN H¹, ¹³C et microanalyse.

### 3 - Les bisgluconamidodioxo ou trioxoalcanes et les bislactobionamidodioxo ou trioxoalcanes

Dans le but de fonctionaliser la chaîne hydrophobe des bolaformes, on a utilisé des diamines à longues chaînes oxygénées.

La formule générale de ces diamines est la suivante :

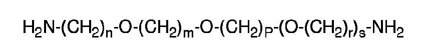

On identifiera les différentes diamines par le code suivant :

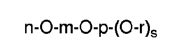

Les diamines oxygénées utilisées seront ainsi :
- la 30203 (_{S}=0)
- la 30603 (₅=0)
- la 3020203 (₅=1, ᵣ=3)

### a) Le bisgluconamidodioxo ou trioxoalcanes

Les différentes amines ont été additionnnées à l'acide gluconique dans les proportions respectives 1/2. Le solvant utilisé diffère selon les diamines employées.

Les conditions opératoires ainsi que les rendements sont rassemblés dans le tableau suivant :

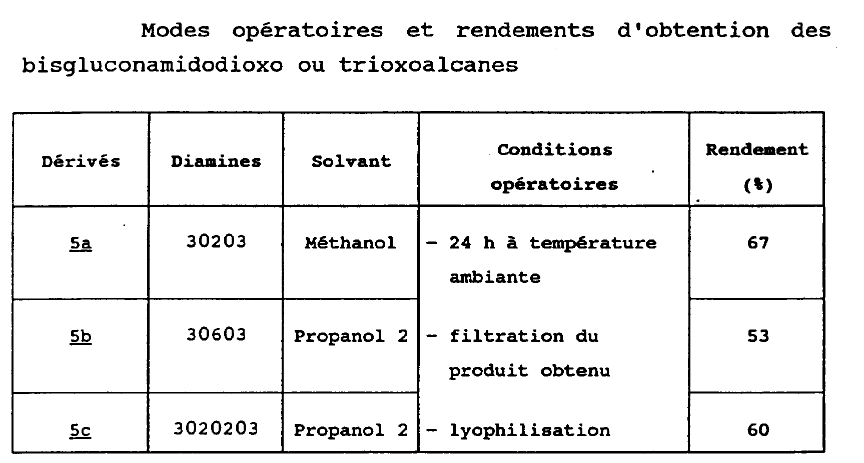

Les produits obtenus se présentent sous forme dune poudre blanche hygroscopique et ont la formule suivante :

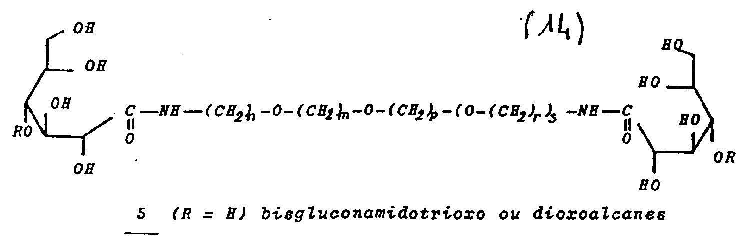

5 (R=H) bisgluconamidotrioxo ou dioxoalcanes

Ils ont été caractérisés par RMN H¹, ¹³C et microanalyse.

### b) Les bislactobionamidodioxo ou trioxoalcanes

L'acide lactobionique en solution aqueuse est additionné à la diamine oxygénée en solution dans le propanol-2.

Comme précédemment, les conditions opératoires varient selon les diamines utilisées. Les conditions opératoires ainsi que les rendements de la réaction sont réunis dans le tableau suivant :

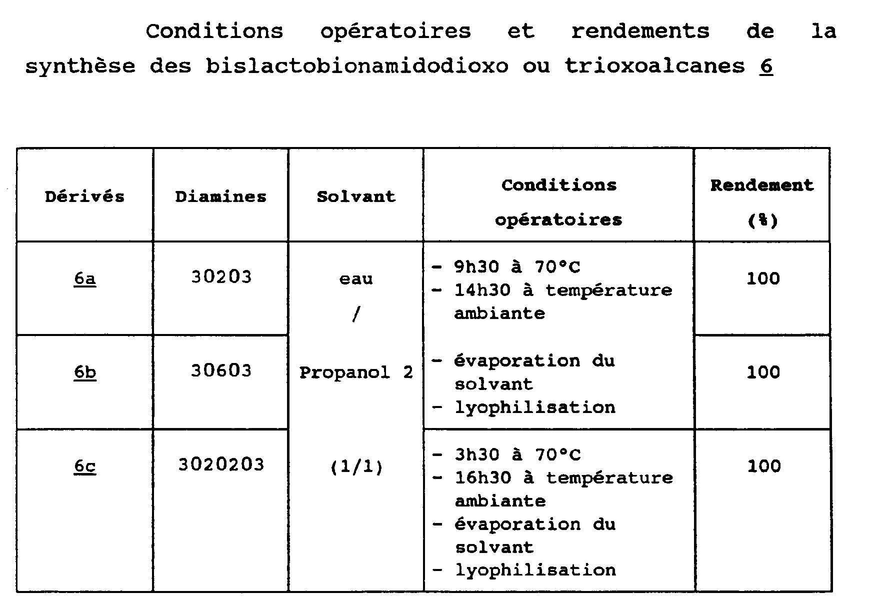

Les bislactobionamidodioxo ou trioxoalcanes ont pour formule la même que précédemment (cf composé 5) mais ici R = galactose (composé 6).

Ces produits sont très hygroscopiques, ils doivent être conservés sous atmosphère inerte. Ils ont été caractérisés par _{R}MN H¹, ¹³C et microanalyse.

### 4 - Les bisaminolactose alcanes

Jusquà présent les composés bolaformes synthétisés comportent un cycle glucose ouvert. Il est apparu intéressant de synthétiser des composés bolaformes dont le cyle glucose reste fermé.

On a donc essayé de synthétiser les bolaformes suivants :

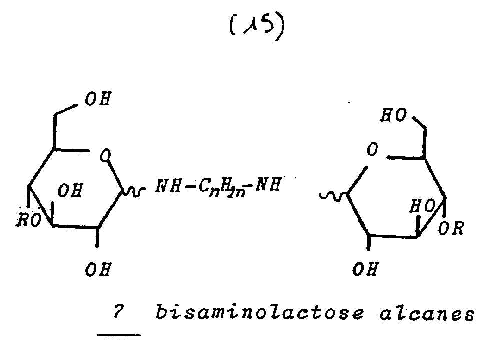

7 bisamonolactose alcanes R = Galactose

La synthèse (n=12) se fait en une étape : on additionne le lactose en solution aqueuse à la diamine en solution dans le propanol-2. La réaction se fait pendant 24 heures. Le produit est purifié par recristallisation. Le rendement de la réaction est de 70 %.

Le produit a été caractérisé par RMN H¹, ¹³C et microanalyse.

### 5 - Bisglucosaminoalcanes

Comme précédemment, l'intérêt de synthétiser des composés bolaformes à partir de la glucosamine est la conservation du cycle pyranosyl et en outre de sa fonction alcool primaire anomérique.

Contrairement à tout ce qui a été fait jusqu'à présent, la fonction amine est portée par le sucre.

On a synthétisé les réactifs acylants bolaformes correspondants.

### a) Synthèse des réactifs acylants

Ils sont synthétisés à partir de la 2 mercaptothiazoline et de dichlorure d'acide à longue chaîne alcane 8 selon le schéma suivant :

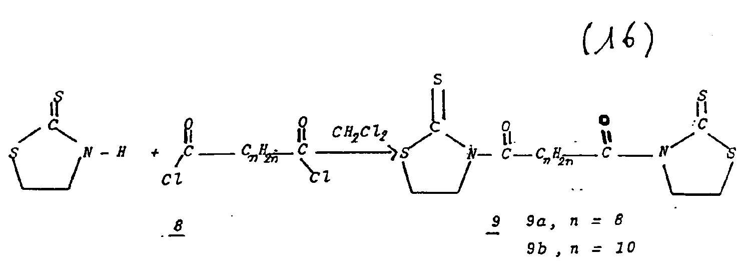

La réaction se fait en présence de triéthylamine dans le dichlorométhane pendant 3 heures.

Les réactifs acylants sont recristallisés et ils se présentent sous forme de poudre fluorescente.

Les rendements de la réaction sont rassemblés dans le tableau suivant :

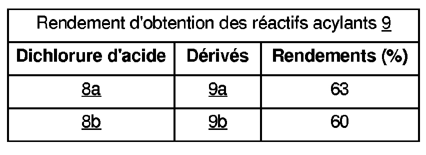

Les réactifs acylants ont été caractérisés par RMN H¹, ¹³C et microanalyse.

### b) Synthèse des bolaformes

La glucosamine est commercialisée sous forme de chlorydrate. Elle est donc mise en solution dans le DMF en présence de triéthylamine. A cette solution, on ajoute le réactif acylant.

Le schéma de la réaction est le suivant :

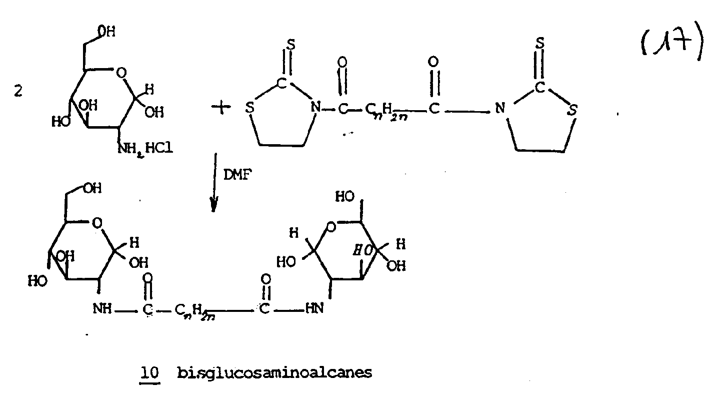

Le mélange réactionnel est alors agité pendant 6h30 à 70°C, puis 17h30 à température ambiante. Le composé bolaforme 10 précipite dans le mélange réactionnel, il est filtré puis lyophilisé.

Les rendements sont réunis dans le tableau suivant :

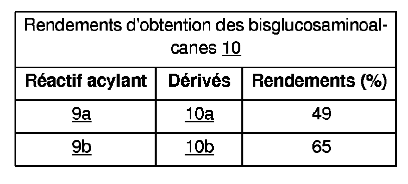

Mode opératoire des réactifs acvlants :

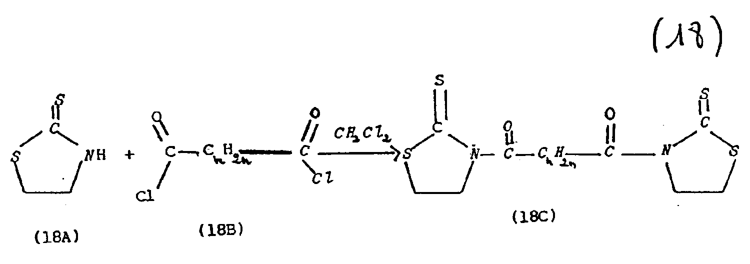

On mélange la thiazoline (35,4 mmol) avec la triéthylamine (32,42 mmol) dans 80 ml de dichlorométhane.

Le dichlorure d'alkyle en solution dans le dichlorométhane (19,25 mmol, 15 ml) est ajouté goutte à goutte au mélange précédent. Après l'addition totale du dichlorure, le mélange réactionnel est laissé sous agitation pendant 3 heures.

Lors de la réaction du dichlorure d'alkyle sur la mercaptothiazoline, la solution devient jaune fluroescente, ce qui caractérise la formation du réactif acylant.

Le mélange réactionnel est alors lavé avec une solution d'acide chlorydrique 0,5N, puis avec une solution de NaHC0₃ à 5 %. La phase organique est alors évaporée et le produit jaune fluorescent est recristallisé dans l'acétoni- trile.

Ces composés ont été caractérisés par microanalyse, RMN H¹ et RMC¹³.

### Mode opératoire des bolaformes :

Le glucosamine chlorydrate (4,65 mmol) est solubilisé dans le diméthylformamide en présence de triéthylamine (5,11 mmol). A cette solution est ajouté le réactif acylant bolaforme (2,35 mmol).

On suit la formation du composé bolaforme en observant la décoloration jaune fluorescente du mélange. Le mélange réactionnel est alors agité pendant 6h30 à 70°C, puis 17h30 à température ambiante.

Il y a formation d'un précipité blanc, selon le schéma réactionnel ci-après, on filtre alors le mélange réactionnel et on sèche le produit.

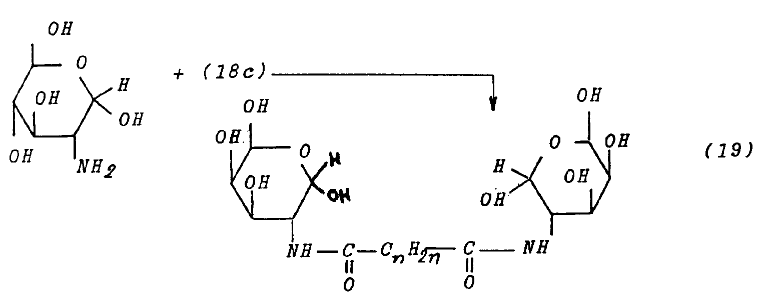

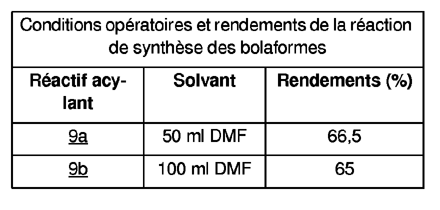

### 1 - Les dilactobioamidospermidines

A une solution contenant 1g d'acide lactobionique (2,7 mmol) et 25 ml d'eau, 25 ml de propanol-2, on ajoute la spermidine (1,35 mmol).

Le milieu réactionnel est agité pendant 24 h à température ambiante.

Le solvant est évaporé et le solide obtenu est lyophilisé.

On obtient 1,2 g de produit hygroscopique. Ce solide blanc a pour formule : 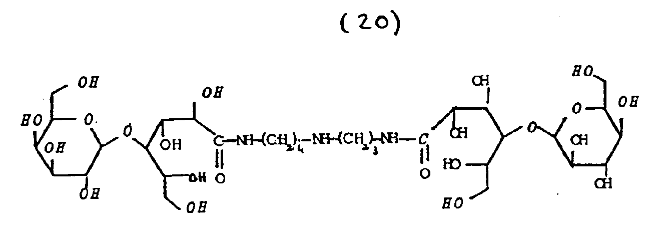
C₃₁ H₅₉ ^{N}₃ 0₂₂
Ces produits ont été caractérisés par microanalyse, RMN H¹ et RMNC¹³.

### Il - Les dilactobioamidospermines

A une solution contenant 1 g d'acide lactobionique (2,7 mmol) et 100ml de méthanol, on ajouté la spermine (1,35 mmol).

Le milieu est agité pendant 24 heures. Le solvant est évaporé et le produit blanc est lyophilisé.

On obtient alors 1,325 g de produit blanc qui est fortement hygroscopique, sa formule développée est la suivante : 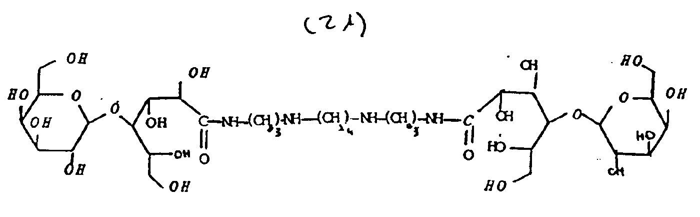
C₃₄ H₆₆ N₄ O₂₂
Ces produits ont été caractérisés par microanalyse, RMNH¹ et RMNC¹³.

### III - Les composés bolaformes bisgluconamidodioxi ou trioxoalcanes

Les diamines à longues chaînes oxygénées ont pour formule :

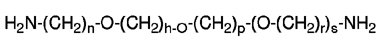

On les identifiera par le code suivant :

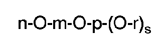

Au mélange contenant l'acide gluconique (5,56 mmol) et 50 ml de solvant, on ajoute la diamine oxygénée (2,65 mmol).

Le milieu est agité pendant 24 heures à température ambiante. Le produit précipite. Il est alors filtré puis lyophilisé.

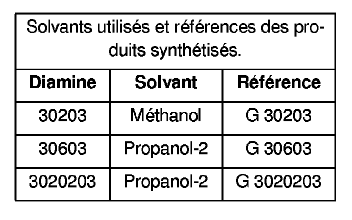

Formule générale des bisgluconamidotrioxo ou dioxoalcanes :

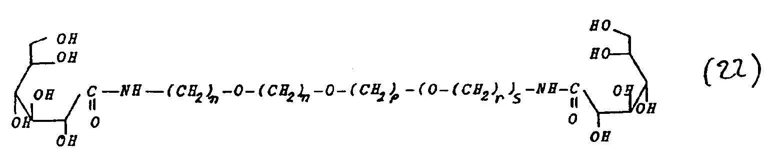

Tous ces produits ont été caractérisés par microanalyse, RMNH¹ et RMNC¹³.

### IV - Les composés bolaformes bislactobionamidotrioxo ou dioxoalcanes

A 1g d'acide lactobionique (2,7 mmol) en solution dans 25 ml d'eau et 25 ml de propanol-2, on ajoute la diamine oxygénée (1,35 mmol).

Le milieu réactionnnel est alors chauffé à 70°C pendant 9h30, puis il est laissé sous agitation pendant 14h30. on évapore le solvant et on lyophilise.

Les produits obtenus sont fortement hygroscopiques et ont pour formule générale suivante :

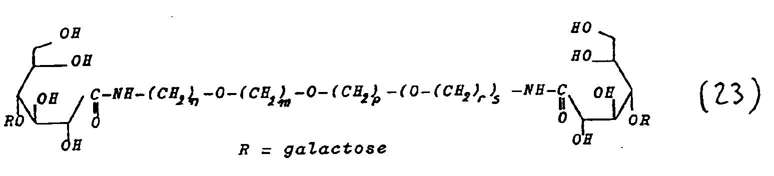

Ils sont caractérisés par microanalyse, RMN H¹ et R_{MNC}¹³.

### V - Les bisaminolactoses alcanes

A une solution de lactose (20,8 mmol) dans 40 ml d'eau est ajouté la diamine (9,9 mmol ) solubilisée dans 20 ml de propanol.

Le mélange est alors agité à température ambiante pendant 24 heures. on chauffe alors à 60°C pendant 30'.

Le solvant est évaporé et le solide est repris à l'éthanol et au toluène pour élimier les traces d'eau. Le produit est purifié par recristallisation dans l'éthanol.

Le rendement de la réaction est de 70%.

### VI - Les dérivés bisgluconamidoalcanes selon formule (1)

Par convention, ces bisgluconamidoalcanes seront désignés par la séquence n-n'-n, représentant le nombre de méthylène.

### a) Préparation du dérivé 6-4-6

A 15 g de s-gluconolactone (84,2 mmole) pesé dans un tricol de 1 litre, on ajoute 750 ml de méthanol, puis 9,8 g de 1.6 diaminohexane (84,2 mmole) et 6,2 g d'acide adipique (42,1 mmole).

Le milieu réactionnel est agité pendant 24 heures à 25°C. Le produit de réaction précipite ; il est récupéré par filtration sur sur buchner puis lavé à chaud par 3 x 100 ml de méthanol à 60°C environ.

Le produit est récupéré dans un cristallin pour y être séché pendant 24 heures dans un dessicateur sous vide. 26,5 g de dérivé 6-4-6 sont récupérés, soit un rendement global de 90 %.

### b) Préparation du dérivé 6-10-6

A 15 g de 5 gluconolactone (84,2 mmole) pesé dans un tricol de 1 litre, on ajoute 750 ml de méthanol, puis 9,8 g de 1.6 diaminohexane (84,2 mmole) et de 9,7 g d'acide dodecanedioïque (42,1 mmole).

Le milieu réactionnel est ajouté pendant 24 heures à 25° C. Le produit de réaction précipité ; il est récupéré par filtration sur buchner, puis lavé à chaud par 3 x 100 ml de méthanol à 60° C environ.

Le produit est récupéré dans un cristallin pour y être séché pendant 24 heures dans un dessicateur sous vide. 29,5 g de dérivé 6-10-6 sont récupérés, soit un rendement global de 89,6 %.

### c) Préparation du dérivé 2-10-2

A 15 g de de 8 gluconolactone (84,2 mmole)pesé dans un tricol de 1 litre, on ajoute 750 ml de méthanol, puis 5 g d'éthylènediamine (84,2 mmole) et de 9,7 g d'acide dodecanedinoïque (42,1 mmole).

Le milieu réactionnel est agité pendant 24 heures à 25° C. Le produit de réaction précipite ; il est récupéré par filtration sur buchner, puis lavé à chaud par 3 x 100 ml de méthanol à 60° C environ.

Le produit est récupéré dans un cristallin pour y être séché pendant 24 heures dans un dessicateur sous vide. 23,4 g de dérivé 2-10-2 sont récupérés soit un rendement global de 83 %.

## Revendications

1. Utilisation d'un composé bolaamphiphile ou bolaforme, répondant à la formule générale suivante :
RAEAR
dans laquelle :
- E représente une chaîne carbonée comportant au moins six atomes de carbone.
- A est un groupe fonctionnel de liaison, et
- R est le résidu d'un polysaccharide réducteur à chaîne linéaire ou cyclisée, ou d'un dérivé dudit polysaccharide, lié directement ou indirectement par une de ses fonctions aldéhyde ou cétone, ou fonction dérivée, au groupe fonctionnel A,
pour obtenir une composition tensioactive.

2. Utilisation selon la revendication 1, caractérisée en ce que la chaîne carbonée E est une chaîne carbonée saturée.

3. Utilisation selon la revendication 1, caractérisée en ce que la chaîne carbonée E comporte de 6 à 24 atomes de carbone, de préférence de 6 à 14 atomes de carbone.

4. Utilisation selon l'une des revendications 1 à 3, caractérisée en ce que la chaîne carbonée E est interrompue par au moins un groupement aromatique, hétérocyclique ou fonctionnel, le groupement fonctionnel étant choisi parmi les fonctions -NH- et -0-.

5. Utilisation selon l'une des revendications précédentes, caractérisée en ce que le groupe fonctionnel A est choisi parmi les fonctions : 0, OCO, NH, NHCO et S.

6. Utilisation selon la revendication 5, caractérisée en ce que le groupe fonctionnel A est NH.

7. Utilisation selon l'une des revendications précédentes, caractérisée en ce que le glucide réducteur formant le résidu R est un monosaccharide.

8. Utilisation selon la revendication 7, caractérisée en ce que le résidu R est choisi parmi le glucose et le galactose.

9. Utilisation selon l'une des revendications précédentes, caractérisée en ce que le glucide réducteur formant le résidu R est un polysaccharide.

10. Utilisation selon la revendication 9, caractérisée en ce que le résidu R est un disaccharide notamment choisi parmi le lactose, le cellobiose et le maltose.

11. Utilisation selon l'une quelconque des revendications précédentes, pour obtenir une composition tensioactive destinée à préparer un agent vésiculaire.

## Claims

1. The utilization of a bola-amphiphilic or bolaform compound complying with the following basic formula:
RAEAR
in which:
- E stands for a carbon chain including at least six carbon atoms,
- A is an active binding group, and
- R is the residue of a straight or ring-chain reducing polysaccharide, or of a derivative of said polysaccharide, bound directly or indirectly by one of its aldehyde or ketone groups, or a derivative group, to the active group A,
so as to obtain a surface-active composition.

2. A utilization according to claim 1, characterised in that the carbon chain E is a saturated carbon chain.

3. A utilization according to claim 1, characterised in that the carbon chain E includes between 6 and 24 carbon atoms, preferably between 6 and 14 carbon atoms.

4. A utilization according to one of claims 1 to 3, characterised in that the carbon chain E is interrupted by at least one aromatic heterocylic or active group, the active group being selected among the -NH- and -0- groups.

5. A utilization according to one of the prior claims, characterised in that the active group A is selected among the following groups: 0, OCO, NH, NHCO and S.

6. A utilization according to claim 5, characterised in that the active group A is NH.

7. A utilization according to one of the prior claims, characterised in that the reducing saccharose forming the residue R is a monosaccharide.

8. A utilization according to claim 7, characterised in that the residue R is selected among saccharose and galactose.

9. A utilization according to one of the prior claims, characterised in that the reducing saccharose forming the residue R is a polysaccharide.

10. A utilization according to claim 9, characterised in that the residue R is a disaccharide particularly selected among lactose, cellose and maltose.

11. A utilization according to any of the prior claims, so as to obtain a surface-active composition intended to prepare a vesicular agent.

## Patentansprüche

1. Anwendung einer bolaamphiphilen oder bolaformen Verbindung mit folgender allgemeinen Formel :
RAEAR
in der
- E eine Kohlenstoffkette darstellt mit mindestens sechs Kohlenstoffatomen,
- A eine funktionnelle Verbindungsgruppe ist, und
- R der Rückstand eines Reduktionspolysaccharids mit gerader oder zyklisierter Kette oder eines Derivats dieses Polysaccharids ist, der durch eine seiner Aldehyd- oder Ketongruppen oder Derivatgruppe direkt oder indirekt mit der Wirkgruppe A verkettet ist,
zur Herstellung einer Tensidzusammensetzung.

2. Anwendung nach Anspruch 1, dadurch gekennzeichnet, daß die Kohlenstoffkette E eine gesättigte Kohlenstoffkette ist.

3. Anwendung nach Anspruch 1, dadurch gekennzeichnet, daß die Kohlenstoffkette E 6 bis 24 Kohlenstoffatome enthält, vorzugsweise 6 bis 14 Kohlenstoffatome.

4. Anwendung nach einem der Ansprüche 1 bis 3, dadurch gekennzeichnet, daß die Kohlenstoffkette E durch mindestens eine aromatische, heterozyklische oder funktionelle Gruppierung unterbrochen ist, wobei die funktionelle Gruppierung aus den Gruppen -NH- und -0- gewählt ist.

5. Anwendung nach einem der vorhergehenden Ansprüche, dadurch gekennzeichnet, daß die funktionnelle Gruppe A aus den Gruppen 0, OCO, NH, NHCO und S gewählt ist.

6. Anwendung nach Anspruch 5, dadurch gekennzeichnet, daß die funktionelle Gruppe A NH ist.

7. Anwendung nach einem der vorhergehenden Ansprüche, dadurch gekennzeichnet, daß das den Rückstand R bildende Reduktionskohlenhydrat ein Monosaccharid ist.

8. Anwendung nach Anspruch 7, dadurch gekennzeichnet, daß der Rückstand R aus Glukose und Galaktose gewählt ist.

9. Anwendung nach einem der vorhergehenden Ansprüche, dadurch gekennzeichnet, daß das den Rückstand R bildende Reduktionskohlenhydrat ein Polysaccharid ist.

10. Anwendung nach Anspruch 9, dadurch gekennzeichnet, daß der Rückstand R ein insbesondere aus Laktose, Zellobiose und Maltose gewähltes Disaccharid ist.

11. Anwendung nach einem der vorhergehenden Ansprüche, zur Herstellung einer Tensidzusammensetzung für die Präparation eines blasenziehenden Mittels.
